Europäisches Patentamt

European Patent Office

Office européen des brevets

(19)

(11) Veröffentlichungsnummer : **0 075 240
B1**

(12) **EUROPÄISCHE PATENTSCHRIFT**

(45) Veröffentlichungstag der Patentschrift :
26.06.85

(21) Anmeldenummer : 82108426.6

(22) Anmeldetag : 13.09.82

(51) Int.- Cl.⁴ : **A 01 N 59/26, A 01 N 59/12//
A61L2/18**

(54) Jodhaltige Desinfektionsmittel.

(30) Priorität : 19.09.81 DE 3137339

(43) Veröffentlichungstag der Anmeldung :
30.03.83 Patentblatt 83/13

(45) Bekanntmachung des Hinweises auf die Patenterteilung : 26.06.85 Patentblatt 85/26

(84) Benannte Vertragsstaaten :
AT BE CH DE FR GB IT LI NL SE

(56) Entgegenhaltungen :
CH-A- 506 295
DE-A- 2 427 702
US-A- 1 903 614
US-A- 3 308 014
Die Akte enthält technische Angaben, die nach dem
Eingang der Anmeldung eingereicht wurden und die
nicht in dieser Patentschrift enthalten sind.

(73) Patentinhaber : Henkel Kommanditgesellschaft auf
Aktien
Postfach 1100 Henkelstrasse 67
D-4000 Düsseldorf-Holthausen (DE)

(72) Erfinder : Schlüssler, Hans-Joachim, Dr.
Am Mühlenbusch 45
D-5667 Haan (DE)
Erfinder : Koch, Ferdinand
Händelstrasse 36
D-4010 Hilden (DE)

**0 075 240**

**Beschreibung**

Es ist bekannt, daß jodhaltige Desinfektionsmittel auf Basis Kalium- oder Natriumjod und elementaren Jods hergestellt und in wäßriger Phase auf dem medizinischen Sektor angewendet werden (Jodtinktur). Die gebräuchlichen Jodlösungen enthalten teilweise neben den oben angegebenen Grundsubstanzen auch noch Calciumjodid sowie unterschiedliche Anteile an Lösungsvermittlern wie beispielsweise Ethanol, Isopropanol oder Glyzerin.

Weiterhin sind in den letzten Jahren Jodprodukte, bestehend aus oberflächenaktiven, anionischen, kationischen, vorzugsweise nichtionischen Substanzen, unter der allgemeinen Bezeichnung Jodophore auf den Markt gebracht worden. Diese Erzeugsnisse werden nicht nur im medizinischen, sondern auch im lebensmittelverarbeitenden Sektor verwendet. Diese Gruppe der Jodophore enthält neben den oben aufgeführten oberflächenaktiven Substanzen und elementarem Jod und Jodiden unterschiedliche Anteile an Phosphorsäure.

Darüber hinaus sind die oberflächenaktiven Substanzen teilweise oder vollständig durch hochpolymere Stoffe wie beispielsweise Polyacrylat oder andere substituierte Polycarbonsäuren ersetzt worden. Die Mittel können ferner verschiedene Anteile an Lösungsvermittlern, wie Isopropanol oder Ethanol, enthalten.

Es hat sich nun gezeigt, daß die Anwendung der wäßrigen beziehungsweise alkoholischen Lösungen, die für den medizinischen Sektor verwendet werden, für die Desinfektion von festen Oberflächen, die nach Reinigungsprozessen wiederum mit Lebensmitteln in Berührung kommen, zu ungünstigen, nicht optimalen Ergebnissen führen. Auch waren die Oberflächen (Kunststoff) nach der Behandlung teilweise bräunlich verfärbt und die Stabilität der Lösung unzureichend, was am Aufhellen der typischen bräunlich-gelben Farbe des elementaren Jods in der wäßrigen Phase zu erkennen war.

Die Gruppe der Jodophore zeigt zwar gute bakteriologische Wirkung. Es haben sich aber auch Nachteile bei der Anwendung ergeben. So ist trotz Verwendung von Schauminhibitoren ein absolut schaumfreies Arbeiten in den Fertigungsanlagen für verschiedene Lebensmittel nicht möglich gewesen. Darüber hinaus belasten die Präparate mit oberflächen- sowie mit nichtoberflächenaktiven, polymeren Verbindungen das Abwasser, indem sie die sogenannten CSB-Werte (chemischer Sauerstoffbedarf) heraufsetzen. Auch hat sich gezeigt, daß die Abspülbarkeit der Jodophore gegenüber Lösungen ohne oberflächenaktive Substanzen ungünstiger einzustufen ist. Ebenso sind Spannungsrißkorrosionen, bedingt durch nichtionogene, ethoxylierte und/oder propoxylierte Blockpolymerisate, die als Jodträger dienten, in Rohrmelkanlagen bei Acrylglas beobachtet worden.

Ferner hat sich als Nachteil erwiesen, daß die Lösungsvermittler, wie zum Beispiel Ethyl- und Isopropylalkohol, nicht nur das Abwasser belasten, sondern außerdem durch ihren verhältnismäßig niedrigen Flammpunkt besondere Vorsichtsmaßnahmen bei der Herstellung (explosionsgeschützte Anlagen) und bei der Verpackung (Entgasungsverschluß) notwendig machen.

Aus der Schweizer Patentschrift 506 295 ist ein Jod und Wasser enthaltendes Desinfektionsmittel bekannt, das aus Jod, Kaliumjodid, einer Mineralsäure oder organischen Säure und Wasser besteht. Wenngleich dieses Mittel recht günstige Eigenschaften aufweist, so ist doch seine Lagerstabilität, d. h. der Prozentsatz freies Jod nach Lagerung, noch nicht befriedigend, da bereits nach ca. 4 Monaten ein Rückgang des freien Jodgehalts zu beobachten ist.

Es wurde nun gefunden, daß man diese Nachteile und Mängel vermeiden kann, wenn man sich der nachstehend beschriebenen jodhaltigen Desinfektionsmittelkonzentrate bedient. Diese bestehen aus

0,1 bis 3 Gew.-%, vorzugsweise 1 bis 2 Gew.-%, $J_2$
0,2 bis 6 Gew.-%, vorzugsweise 2 bis 4 Gew.-%, Kalium- oder Natriumjodid
1 bis 15 Gew.-% Phosphorsäure
1 bis 3 Gew.-% Phosphonsäure mit komplexierenden Eigenschaften
Rest Wasser.

Als Phosphonsäure mit komplexierenden Eigenschaften kommen 1-Hydroxyalkan-1,1-diphosphonsäure, wie 1-Hydroxyethan-1,1-diphosphonsäure, 1-Aminoalkan-1,1-diphosphonsäure, wie 1-Aminoethan-1,1-diphosphonsäure sowie vorzugsweise Aminotrimethylenphosphonsäure in Betracht.

Die erfindungsgemäßen Mittel enthalten somit keine Netzmittel, keine Jodophore und keine nichtoberflächenaktiven Polymer-Verbindungen. Ebenfalls sind sie frei von Lösungsvermittlern, wie niedere ein- oder zweiwertige Alkohole.

Aus diesen Konzentraten werden Einsatzlösungen in vorzugsweise 0,5 bis 2 %iger Konzentration in Wasser hergestellt.

Die Konzentrate sind auch noch nach einem Jahr stabil und zeigen keinen Abfall des Aktivjodgehaltes. Auch sind keine Abscheidungen elementaren Jods im Inneren der Aufbewahrungsgefäße zu beobachten.

Die bakteriologischen Ergebnisse zeigen ferner im Vergleich zu den typischen Jodophoren keinen Unterschied, und darüber hinaus sind die beanspruchten Gemische preisgünstiger herzustellen.

Der Anmeldungsgegenstand wird noch durch die nachstehenden Beispiele erläutert, dabei stellt die mit A) bezeichnete Rezeptur Vergleichsprodukte dar.

2

**0 075 240**

Beispiel

Aus den Jodkonzentraten A und B, bestehend aus

A) 1 Gew.-% Jod      (Vergleichsprodukt)
   2 Gew.-% Natriumjodid
   6 Gew.-% Polyacrylsäure
   60 Gew.-% Phosphorsäure
   15 Gew.-% Ethylalkohol
   16 Gew.-% dest. Wasser

B) 1 Gew.-% Jod
   2 Gew.-% Natriumjodid
   12 Gew.-% Phosphorsäure
   1 Gew.-% Nitrilotrimethylenphosphonsäure
   84 Gew.-% dest. Wasser

wurden Verdünnungen in 1,5 %iger Konzentration hergestellt und in einer Molkerei zur Desinfektion von Milchtanks und Rohrleitungssystemen nach dem Reinigungsvorgang eingesetzt. Die Lösungen wurden 3 Tage verwendet und vor dem Ablassen in das Abwassersiel auf ihren CSB-wert (chemischer Sauerstoffbedarf in mg/l) überprüft. Die Summe CSB, bestehend aus geringfügigen Schmutzmengen und der Lösung A), wurde mit 2 400 mg/l $O_2$ ermittelt.

Der unter gleichen Belastungsbedingungen gereinigte und gespülte Tank nebst Leitungssystem wurde nachfolgend mit Lösung B) desinfiziert, bei der nach 3 Tagen nur ein CSB von 1 200 mg/l $O_2$ festgestellt wurde.

**Patentanspruch**

Jodhaltige Desinfektionsmittelkonzentrate, bestehend aus

0,1 bis  3 Gew.-%, vorzugsweise 1 bis 2 Gew.-%, $j_2$
0,2 bis  6 Gew.-%, vorzugsweise 2 bis 4 Gew.-%, Kalium- oder Natriumjodid
1   bis 15 Gew.-% Phosphorsäure
1   bis  3 Gew.-% Phosphonsäure mit komplexierenden Eigenschaften, wie 1-Hydroxyalkan-1,1-diphosphonsäure, 1-Aminoalkan-1,1-diphosphonsäure oder Aminotrimethylenphosphonsäure
Rest Wasser.

**Claim**

Iodine-containing disinfectant concentrates consisting of

from 0.1 to  3 % by weight and preferably from 1 to 2 % by weight of $I_2$,
from 0.2 to  6 % by weight and preferably from 2 to 4 % by weight of potassium or sodium iodide,
from 1    to 15 % by weight of phosphoric acid,
from 1    to  3 % by weight of phosphonic acid having complexing properties, such as 1-hydroxyalkane-1,1-diphosphonic acid, 1-aminoalkane-1,1-diphosphonic acid or aminotrimethylene phosphonic acid,
remainder water.

**Revendication**

Concentré de désinfectant contenant de l'iode, constitué par :

0,1 à  3 % en poids, de préférence 1 à 2 % en poids de $I_2$,
0,2 à  6 % en poids, de préférence 2 à 4 % en poids d'iodure de potassium ou de sodium,
1   à 15 % en poids d'acide phosphorique,
1   à  3 % en poids d'acide phosphonique ayant des propriétés complexantes, tel que les acides 1-hydroxyalcane-1,1-diphosphonique, 1-aminoalcane-1,1-diphosphonique ou amino-triméthylènephosphonique,
le reste étant de l'eau.